# EUROPEAN PATENT SPECIFICATION

(11) **EP 0 683 038 B1**
(45) Date of publication and mention of the grant of the patent: **29.08.2001**
(21) Application number: 95303371.9
(22) Date of filing: 19.05.1995
(51) Int. Cl.: B32B 27/08, A61F 5/445, C23C 14/10, C23C 14/20

(54) **Ostomy pouch film construction**
Filmstruktur für Ostomiebeutel
Structure de film pour poche d'ostomie

(30) Priority: 19.05.1994 GB 9410059; 14.07.1994 GB 9414268
(43) Date of publication of application: 22.11.1995
(73) Proprietor: E.R. SQUIBB & SONS, INC., Princeton, New Jersey 08543-4000 (US)
(72) Inventor: Steer, Graham Emery, Fulham, London SW6 6AF (GB); Gent, John A., Liphook, Hampshire GU30 7AJ (GB)
(74) Representative: Holmes, Miles Keeton

(56) References cited:
- EP-A- 0 023 389
- EP-A- 0 034 392
- DE-A- 2 719 113
- GB-A- 2 201 372
- US-A- 4 309 466
- US-A- 4 661 395
- DATABASE WPI Section Ch, Week 7447 Derwent Publications Ltd., London, GB; Class A32, AN 74-81463V & JP-A-49 034 984 ( UNITIKA LTD) , 30 March 1974
- DATABASE WPI Section Ch, Week 8140 Derwent Publications Ltd., London, GB; Class A35, AN 81-72648D & JP-A-56 104 022 ( ITO S) , 19 August 1981

## Description

This invention relates to a film for making ostomy pouches, and a method of making such a film.

The film used for the production of ostomy pouches has to possess certain properties for the end-product to meet functional requirements and patient (wearer) acceptance. These properties include:-
1. Mechanical strength such that the pouch does not fail in service due to tearing, creep, etc.
2. The film must be weldable in order to form a pouch. The resultant bond must have adequate strength to ensure that the pouch does not burst or leak during usage.
3. It must provide an adequate barrier against odour emanating from body waste. These gases can escape by transmission through various known types of film, whereas the preferred route is to pass them through a deodorising filter.
4. It must be comfortable for the patient to wear, and to be essentially free of rustle characteristics when the patient moves or sits.

Laminated and co-extruded films meeting the above criteria have been developed over a number of years. A polymer which often has been utilised to provide the desired gas barrier properties of the aforesaid films is a copolymer of polyvinylidene chloride. However, in recent times there has been a move towards environmental improvements, and it is therefore a desire to eliminate chlorine-containing polymers from the pouch films. It would also be desirable to achieve a less expensive film. There are alternative polymers which are capable of providing an adequate gas barrier when the moisture content of the polymer is low. Examples include polyvinyl alcohols (PVOH), ethylene vinyl alcohols (EVOH) and polyamides (nylons). When an ostomy pouch is used it is subjected to moisture on the inside from body waste, and on the outside there is sweat. Also, when worn, the temperature of the bag approaches that of the body and these three factors when combined have the effect of reducing the performance of the bag in terms of barrier properties to an unacceptable level. This is a particular problem when the bag is worn for extended periods.

Examples of materials which have virtually perfect gas barrier properties are metals and glass. However, unless these materials are exceptionally thin, that is to say of a thickness of about a micron or less, they would be too rigid, and hence not meet the other requirements for an ostomy pouch, notably the comfort and noise aspects. The use of vacuum-deposited aluminium as one of the layers in an ostomy pouch has been suggested in British Patent Application No. (GB-A) 2 201 372. By depositing very thin films of metals or glasses onto a smooth polymer film surface, one can obtain products having excellent gas barrier characteristics. Aluminium or glass has been used for this purpose. In packaging applications, but not in ostomy film laminates, one smooth film used is polyester. A disadvantage of the so-called metallised polyester as manufactured for packaging of foods etc., is that the composite films made in this way are unacceptable because they are transparent, or are either too rigid or too noisy to make them acceptable for use in an ostomy pouch. These problems are overcome according to the present invention by using a thin deposited layer of a material (metal or glass, usually metal) possessing excellent barrier characteristics incorporated in a novel manner into a film structure and subsequently used to form an ostomy pouch.

A metallised composite structure has been disclosed in British published application (GB-A) No. 2,069,409 but this is completely unsuitable for an ostomy pouch.

In one aspect, the invention provides a method of making an ostomy pouch wall in the form of a film laminate comprising laminating together a plurality of layers of which at least one is a soft, opaque, flexible, heat-or RF-weldable plastics film up to 70 microns thick, vapour-depositing a metal or glass layer on a smooth polymer film bearing a thin coating layer of lacquer or lacquer-like material, joining the said plurality of layers to the metal or glass layer, removing the smooth polymer film and replacing it by a further laminate which includes at least one plastics film layer.

In a particular embodiment of the invention, a film laminate for use as an ostomy pouch wall is made by (i) applying liquid lacquer as a thin coating layer onto a smooth polymeric film such as polyester, (ii) applying on top of said coating layer a thin (thickness less than about 0.5 microns) coherent film of metal or glass,
(iii) applying thereto one or more layers of plastic film of which at least one is a film as defined herein as Layer A, (iv) separating the smooth polymer film from the laminate formed in step (iii), and (v) uniting the exposed surface of the resulting laminate with a further Layer A as defined herein.

An advantage of the manufacturing procedure described herein is that the integrity of the thin metal or glass film, and hence its gas barrier property, is preserved by depositing it on a plastic film of great smoothness bearing a thin coating layer of a lacquer or a lacquer-like material The step of peeling off, or otherwise removing the smooth polymer film and replacing it with e.g. an e.v.a. film, is advantageous because polyester films and other films of equivalent smoothness, cannot be satisfactorily heat or RF welded to each other.

The present invention aims to use the barrier properties capable of being achieved with a thin coating of either a metal (e.g. aluminium) or glass. In combination with other coatings and adhesives, this thin metal or glass layer is bonded between two thicker polymeric films. Such films may themselves be laminates. The laminated film will then possess both excellent barrier properties and with the correct choice of polymer layers as set out below, be soft, weldable, opaque, flexible, mechanically strong, and essentially rustle-free.

According to one embodiment of the invention, there is provided a film laminate for use in an ostomy pouch, the laminate comprising at least four layers, herein called layers A, B, C and D, whose compositions and characteristics are as set out below.

### Layer A

An opaque thermoplastic film which is capable of being welded to itself using, for example RF or impulse bonding techniques;
- has sufficient strength to support a load of 2kg per 15cm width;
- is soft and flexible;
- shall produce a noise less than that exhibited by a Wall Street Journal when rustled at ambient temperature and humidity; said film being made from any one or more of:

- ethylene vinyl acetate having a vinyl acetate content of 5 to 15% by weight;
- ethylene acrylic acid copolymer;
- polyethylene;

Polyethylene co-polymers with:
- vinyl acetate
- ethyl acrylic acid
- methacrylic acid
- polybutenes
- polyurethane
- polyvinyl chloride [plasticised].
The polymer film may have a thickness of up to 70 microns, preferably 20 to 60 microns.

### Layer B

A flexible adhesive suitable for bonding layers A and C together; e.g. a polyurethane adhesive. This preferably has a thickness of 0.4 to 10 and more preferably 0.5 to 8 microns.

### Layer C

A thin vapour-deposited coating of metal, e.g. aluminium, or glass e.g. silicon dioxide constituting a gas barrier and having a thickness not exceeding 0.15 micron and such that when creased or folded the oxygen transmission rate therethrough is not more than 40ml per square metre per 24 hours at 38°C and at 95 % relative humidity (RH). More preferably, the oxygen transmission rate should be less than 20 ml per square metre per 24 hours at the same temperature and RH.

### Layer D

A thin coating layer, e.g. a lacquer, designed to readily release the bond between itself and a subjacent layer. The thickness of the thin coating layer may be 0.75 to 6 and more preferably 1 to 5 microns.

According to another aspect of the invention, two further layers, one called Layer E and defined below, and the other being Layer A as herein defined, may be added, yielding a laminate having six layers A, B, C, D, E, A. Such a laminate makes a particularly satisfactory wall for an ostomy pouch.

### Layer E

A flexible adhesive suitable for bonding together layers D and A, e.g. a polyurethane adhesive. The thickness of this is preferably as stated above under "Layer B".

The following is a summary of the important features of a preferred method of making a film for an ostomy pouch. This comprises the steps of:
providing a smooth layer of a polyester to form a temporary substrate;
coating Layer D thereonto;
coating Layer C onto layer D;
coating Layer B onto Layer C;
placing the resulting laminate onto Layer A material, so providing a five-layer laminate; then peeling off the polyester and attaching instead Layer A material so achieving a transfer of the A-D laminate from the polyester substrate to a substrate (Layer A) thereby forming a laminated film which is weldable to other components such as an ostomy coupling flange or a deodorising filter.

One example of a manufacturing method according to a preferred embodiment of the invention will be better understood from the following description, given with reference to the accompanying drawings, in which:-
Figure 1 is a diagrammatic illustration of a smooth polymer film (SPF) upon one surface of which has been placed Layer D, that is, a coating layer, for example a lacquer, designed to readily release the bond between itself and the layer SPF. Layer D is cast onto the smooth polymer film using a suitable technique such as gravure, reverse roll coating, or other known method;
Figures 2-6 inclusive are further diagrammatic illustrations of stages in the method.

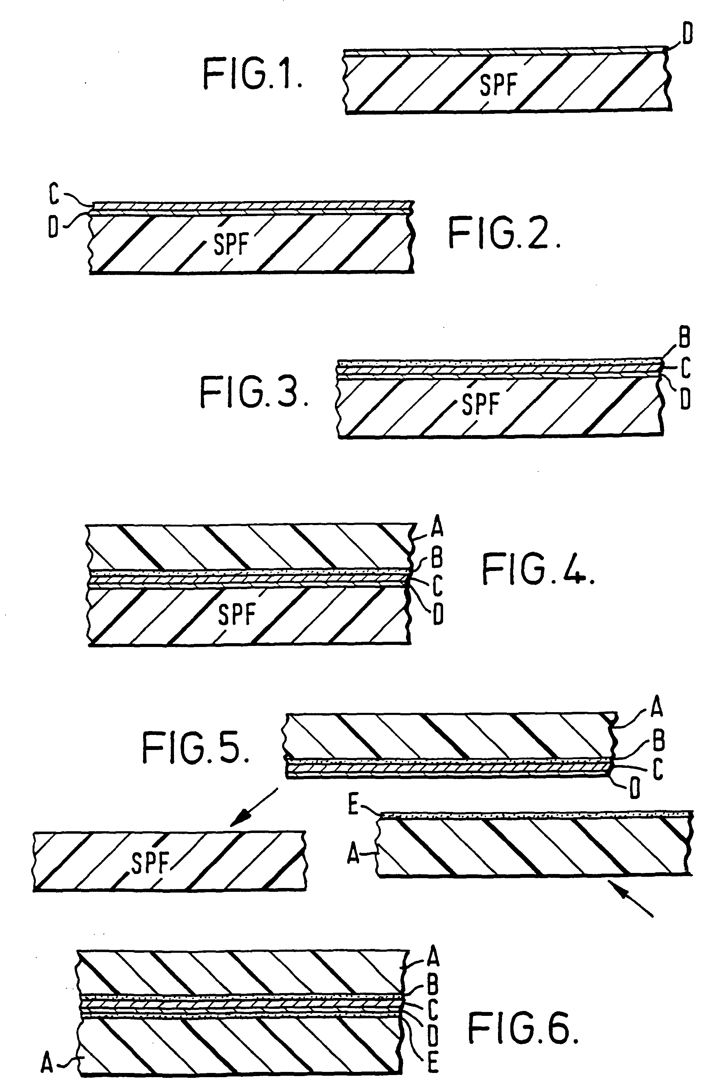

Onto the Layer D there is deposited using a known vacuum deposition method, a thin coating layer C of a metal e.g. aluminium or glass e.g. silicon dioxide. As seen in Figure 3, onto layer C is applied a layer B of adhesive to provide a bond to a layer A which is added to form a five-layer laminate as seen in Figure 4. The next step, seen in Figure 5, is to peel the layer of smooth polymer film (SPF) off the lacquer layer D and bring into contact with the exposed surface of the layer D a separate laminate comprising Layer A as defined above and Layer E, also as defined above. The resulting six-layer laminate is seen in Figure 6.

The thicknesses of the layers may be vaired. Layer A for example may have a thickness up to about 80 microns, and preferably 20 to 60 microns. Layer B may be within the range 0.5 to 8 and more preferably 2 to 2.5 microns in thickness, Layer C preferably about 0.1 micron, Layer D 0.75 to 6 and more preferably 1 to 5 microns, and Layer E 0.5 to 8 and preferably 1.8 to 2.3 microns. It is important to employ a smooth polymer film, preferably polyester, as a temporary substrate, in order to achieve the desired ready separation between the SPF layer and the Layer D.

In a preferred embodiment of the invention, the ostomy pouch film described may have only four layers, namely the four Layers A, B, C and D as shown in the upper part of Figure 5. In this event, the thickness of Layer A would be about 50 to 70 microns. This particular form of film would be useful either if it were required that only one surface of the film need be heat weldable by a plastics welding tool, and/or the nature of Layer D was suitable and adequate to serve as a support to which is attached an ostomy pouch filter. Conventional processes used to prepare Layer A include extrusion and casting.

As will be understood, Layer D is a smooth, thin coating which has some adhesion to the SPF. After Layers A, B, C and D are added thereto as seen in Figure 5, the resulting four-layer laminate is capable of being separated as a unit from the smooth polymer film; that is, the smooth polymer film is peeled off from Layer D, as seen in the left-hand part of Figure 5. As mentioned, the preferred material for the SPF is polyester. It is preferred that the surface of layer D is receptive to printing thereon.

It will be appreciated that the thicknesses of the components in the laminated film are such that it has adequate strength in terms of mechanical properties. For example, in a film laminate as shown in the upper part of Figure 5, Layer A would be at least about 60 microns thick whereas for a laminated film as shown in Figure 6, each of the layers A might be about 30 microns thick.

The polymer which constitutes Layer A could be made by a conventional method, in which the polymer is first melted and fed to an orifice by means of a screw mechanism. It is then fed through a die which may for example be a slot die. The film from the slot die is then passed over a chill roll and is then in its cast form.

The coating (Layer D) is deposited as a liquid and is then changed from a liquid to a solid by, e.g.
- removal of solvent by heating;
- removal of water in an emulsion by heating;
- by cross linking by heat or radiation (e.g. electron beam, ultra violet).
The coating is such as to have a low bond strength to the substrate (SPF) and yet can be removed by peeling. The coating may usefully also have properties of receiving printing and being heat sealable.

A feature of this invention is the temporary use during the manufacturing process of a smooth polymer film to receive the layers D, C, B and A. If, for example, an extruded film were to be used instead, this would be found to have too rough a surface and hence, after layer C was applied, would yield a product with inferior properties, (e.g. tendency to cracks, discontinuities) compared to the quality of composite laminate achieved with a temporary substrate constituted by a smooth polymer film such as polyester. If an extruded film were to be used a substantial coating would have to be applied.

It will be realised that there has been disclosed herein a novel and useful laminated film for use in making an ostomy pouch, and a method for making same.

While the present disclosure has referred to the preferred materials, thicknesses, and manufacturing steps to be employed, it will be appreciated that in the alternative, other materials, thicknesses, and manufacturing steps may be employed.

## Claims

1. An ostomy pouch film which comprises a laminate of
a) a soft, opaque, flexible, heat or RF weldable plastics film (A) not more than about 80 microns thick,
b) an adhesive (B),
c) a vapour-deposited metal or glass layer (C) not exceeding 0.15 microns in thickness, and
d) a thin coating layer (D) of lacquer or a lacquer-like material which has been applied as a liquid.

2. An ostomy pouch film according to claim 1 which further comprises
e) an adhesive layer (E), and
f) a heat or RF weldable plastics film (A).

3. An ostomy pouch film according to Claim 2 in which the film (A) referred to in (f) is the same as that (A) referred to in (a).

4. An ostomy pouch film according to Claim 1, 2 or 3 in which the adhesive (b) and/or (e) is a polyurethane adhesive, and the layer referred to in (d) is a lacquer.

5. An ostomy pouch film according to any one of Claims 1 to 4, in which the said thin coating layer (D) is a film of lacquer of a thickness of 1 to 5 microns.

6. An ostomy pouch film according to any preceding claim in which the thickness of the film (a) is in the range 20 to 60 microns; that of the adhesive (b) is in the range of 0.5 to 8 microns; and that of the adhesive (e) (if present) is in the range 0.5 to 8 microns.

7. An ostomy pouch film according to any preceding claim in which the thickness of the vapour-deposited metal or glass (C) is substantially one micron.

8. An ostomy pouch film according to claim 2 or any claim dependent thereon in which the adhesive (b) or (e) is polyurethane adhesive.

9. An ostomy pouch film according to any one of claims 1-6 in which the vapour-deposited layer (C) is of aluminium and is from 0.05 to 0.1 micron thick.

10. An ostomy pouch film according to any preceding claim in which the layer (c) is vapour-deposited aluminium.

11. An ostomy pouch film according to claim 3 in which the plastics film layer (f) has a thickness in the range 20 to 60 microns.

12. A method of making an ostomy pouch wall in the form of a film laminate (A,B,C,D,E,A) comprising laminating together a plurality of layers (A,B) of which at least one is a soft, opaque, flexible, heat- or RF-weldable plastics film (A) up to 70 microns thick, vapour-depositing a metal or glass layer (C) on a smooth polymer film. (SPF) bearing a thin coating layer (D) of lacquer or lacquer-like material, joining the said plurality of layers to the metal or glass layer (C), removing the smooth polymer film (SPF) and replacing it by a further laminate (E,A) which includes at least one plastics film layer (A).

13. A method according to claim 11 in which the smooth polymer film is polyester and is employed as a temporary substrate.

14. A method according to claim 11 or 12 in which the layers employed are layers (a) to (f) as defined above in claims 1 and 2.

15. A method according to claim13 in which the thicknesses of the layers (a) to (f) are as defined in claims 1, 5, 6, 7, 9 and 11 above.

## Patentansprüche

1. Ostomiebeutelfilm, der einen Schichtstoff aus
a) einem weichen, undurchsichtigen, schmiegsamen, heiss- oder hf-verschweissbaren Kunststofffilm (A), nicht dicker als etwa 80 µm,
b) einen Kleber (B),
c) eine aufgedampfte Metall- oder Glasschicht (C), in ihrer Dicke 0,15 µm nicht übersteigend, und
d) eine dünne Beschichtung (D) aus Lack oder lackartigem Material, das als eine Flüssigkeit aufgetragen worden ist,
umfasst.

2. Ostomiebeutelfilm nach Anspruch 1, der weiterhin
e) einen Kleberfilm (E) und
f) einen heiss- oder hf-verschweissbaren Kunststofffilm (A)
umfasst.

3. Ostosmiebeutelfilm nach Anspruch 2, in dem der in (f) erwähnte Film (A) derselbe wie der in (a) erwähnte (A) ist.

4. Ostomiebeutelfilm nach Anspruch 1, 2 oder 3, in dem der Kleber (b) und/oder (e) ein Polyurethankleber und die in (d) erwähnte Schicht ein Lack ist.

5. Ostomiebeutelfilm nach irgend einem der Ansprüche 1 bis 4, in dem die benannte dünne Beschichtung (D) ein Lackfilm einer Dicke von 1 bis 5 um ist.

6. Ostomiebeutelfilm nach irgend einem vorangehenden Anspruch, in dem die Dicke des Films (a) im Bereich von 20 bis 60 µm; die des Klebers (b) im Bereich von 0,5 bis 8 µm; und die des Klebers (e) (sofern vorhanden) im Bereich von 0,5 bis 8 µm liegt.

7. Ostomiebeutelfilm nach irgend einem vorangehenden Anspruch, in dem die Dicke des aufgedampften Metalls oder Glases (C) im wesentlichen 1 µm beträgt.

8. Ostomiebeutelfilm nach Anspruch 2 oder irgend einem davon abhängigen Anspruch, in dem der Kleber (b) oder (e) ein Polyurethankleber ist.

9. Ostomiebeutelfilm nach irgend einem der Ansprüche 1 bis 6, in dem die aufgedampfte Schicht (C) aus Aluminium besteht und von 0,05 bis 0,1 µm dick ist.

10. Ostomiebeutelfilm nach irgend einem vorangehenden Anspruch, in dem die Schicht (c) aufgedampftes Aluminium ist.

11. Ostomiebeutelfilm nach Anspruch 3, in dem die Kunststofffilmschicht (f) eine Dicke im Bereich von 20 bis 60 µm hat.

12. Verfahren zur Herstellung einer Ostomiebeutelwandung in Gestalt eines Filmschichtstoffs (A,B,C,D,E,A), darin bestehend, eine Mehrzahl von Schichten (A,B) zusammenzufügen, von denen mindestens eine ein weicher, undurchsichtiger, schmiegsamer, heiss- oder hf-verschweissbarer, bis zu 70 µm dicker Kunststofffilm (A) ist, eine Metall- oder Glasschicht (C) auf einen glatten Polymerfilm (SPF) aufzudampfen, der eine dünne Beschichtung (D) aus Lack oder lackartigem Material trägt, die benannte Mehrzahl von Schichten mit der Metall- oder Glasschicht (C) zu verbinden, den glatten Polymerfilm (SPF) zu entfernen und ihn durch einen weiteren Schichtstoff (E,A) zu ersetzen, der mindestens eine Kunststofffilmschicht (A) einschliesst.

13. Verfahren nach Anspruch 11, in dem der glatte Polymerfilm ein Polyester ist und als ein zeitweiliges Substrat verwendet wird.

14. Verfahren nach Anspruch 11 oder 12, in dem die verwendeten Schichten die wie oben in Ansprüchen 1 und 2 definierten Schichten (a) bis (f) sind.

15. Verfahren nach Anspruch 13, in dem die Dicken der Schichten (a) bis (f) wie in Ansprüchen 1, 5, 6, 7 und 11 oben definiert sind.

## Revendications

1. Feuille mince de poche d'ostomie qui comprend un stratifié composé
a) d'une feuille mince (A) en plastique doux, opaque, souple, thermosoudable ou soudable à haute fréquence, d'épaisseur inférieure à environ 80 microns,
b) d'un adhésif (B),
c) d'une couche (C) de verre ou de métal déposé par évaporation sous vide, d'épaisseur ne dépassant pas 0,15 microns, et
d) d'une couche mince de revêtement (D) constituée d'un vernis-laque ou d'un matériau semblable à un vernis-laque qui a été appliqué en phase liquide.

2. Feuille mince de poche d'ostomie selon la revendication 1, qui comprend de plus :
e) une couche adhésive (E), et
f) une feuille mince (A) en plastique thermosoudable ou soudable à haute fréquence.

3. Feuille mince de poche d'ostomie selon la revendication 2, dans laquelle la feuille mince (A) à laquelle on fait référence en (f) est la même que celle (A) à laquelle on fait référence en (a).

4. Feuille mince de poche d'ostomie selon la revendication 1, 2 ou 3, dans laquelle l'adhésif (b) et/ou (e) est un adhésif polyuréthanne, et la couche à laquelle on fait référence en (d) est un vernis-laque.

5. Feuille mince de poche d'ostomie selon l'une quelconque des revendications 1 à 4, dans laquelle ladite couche mince de revêtement (D) est une feuille mince de vernis-laque d'une épaisseur allant de 1 à 5 microns.

6. Feuille mince de poche d'ostomie selon l'une quelconque des revendications précédentes, dans laquelle l'épaisseur de la feuille mince (a) est dans la plage allant de 20 à 60 microns ; celle de l'adhésif (b) est dans la plage allant de 0,5 à 8 microns ; et celle de l'adhésif (e) (s'il est présent) est dans la plage allant de 0,5 à 8 microns.

7. Feuille mince de poche d'ostomie selon l'une quelconque des revendications précédentes, dans laquelle l'épaisseur du métal ou du verre (C) déposé par évaporation sous vide est sensiblement de un micron.

8. Feuille mince de poche d'ostomie selon la revendication 2 ou l'une quelconque des revendications dépendant de celle-ci, dans laquelle l'adhésif (b) ou(e) est un adhésif polyuréthanne.

9. Feuille mince de poche d'ostomie selon l'une quelconque des revendications 1 à 6, dans laquelle la couche (C) déposée par évaporation sous vide est constituée d'aluminium et son épaisseur va de 0,05 à 0,1 micron.

10. Feuille mince de poche d'ostomie selon l'une quelconque des revendications précédentes, dans laquelle la couche (c) est constituée d'aluminium déposé par évaporation sous vide.

11. Feuille mince de poche d'ostomie selon la revendication 3, dans laquelle la couche de feuille mince en plastique (f) présente une épaisseur dans la plage allant de 20 à 60 microns.

12. Procédé de fabrication d'une paroi de poche d'ostomie sous la forme d'un stratifié de feuilles minces (A, B, C, D, E, A) comprenant les étapes consistant à stratifier ensemble une pluralité de couches (A, B) dont l'une au moins est une feuille mince (A) en plastique doux, opaque, souple, thermosoudable ou soudable à haute fréquence, d'épaisseur allant jusqu'à 70 microns, déposer par évaporation sous vide une couche (C) de métal ou de verre sur une feuille mince de polymère lisse (SPF) portant une couche mince de revêtement (D) de vernis-laque ou d'un matériau semblable à un vernis-laque, joindre ladite pluralité de couches à la couche de métal ou de verre (C), enlever la feuille mince de polymère lisse (SPF) et remplacer celle-ci par un autre stratifié (E, A) qui inclut au moins une couche (A) de feuille mince en plastique.

13. Procédé selon la revendication 11, dans lequel la feuille mince de polymère lisse est constituée de polyester et elle est utilisée en tant que substrat temporaire.

14. Procédé selon la revendication 11 ou la revendication 12, dans lequel les couches utilisées sont les couches (a) à (f) telles que définies ci-dessus dans les revendications 1 et 2.

15. Procédé selon la revendication 13, dans lequel les épaisseurs des couches (a) à (f) sont telles que définies dans les revendications 1, 5, 6, 7, 9 et 11 ci-dessus.
